# EUROPÄISCHE PATENTANMELDUNG

(11) **EP 1 038 527 A1**
(43) Veröffentlichungstag der Anmeldung: **27.09.2000**
(21) Anmeldenummer: 00104064.1
(22) Anmeldetag: 28.02.2000
(51) Int. Cl.: A61K 31/525, A61K 9/16

(54) **Verfahren zur Herstellung von Riboflavin-haltigen Granulaten**

(30) Priorität: 24.03.1999 US 275107
(71) Anmelder: BASF AKTIENGESELLSCHAFT, 67056 Ludwigshafen (DE)
(72) Erfinder: Harz, Hans-Peter Dr., 67373 Dudenhofen (DE); Schweikert, Loni Dr., 67122 Altrip (DE); Douglas, Norbert Schmidt, Grosse Ile, MI 48138 (US)

(57) **Zusammenfassung**

Verfahren zur Herstellung von Riboflavingranulaten oder Riboflavinmikrogranulaten mit einem Gehalt an Riboflavin von 90 bis 99,5 Gew.-% und mit einem Partikelgrößenbereich von 50 bis 450 µm, wobei man bei der Granulierung mindestens einen Hilfsstoff, ausgewählt aus der Gruppe, bestehend aus Alkali- und Erdalkalihalogeniden, Alkali- und Erdalkalicarbonaten, Alkali- und Erdalkalihydrogencarbonaten, Alkali- und Erdalkaliphosphaten, quervernetzter Cellulose und Cellulosederivate und quervernetztem Polyvinylpyrrolidon in Mengen von 0,5 bis 10 Gew.-%, alle Gew.-%-Angaben jeweils bezogen auf das Trockenprodukt, zusetzt.

## Beschreibung

Die Erfindung betrifft ein Verfahren zur Herstellung von Riboflavin-haltigen Granulaten.

Riboflavin (Vitamin B₂) wird vielseitig verwendet in der Lebensmittel- und Pharmaindustrie als wesentlicher oder auch nur als färbender Zusatz zu Nahrungsmitteln und Pharmaka. Es fällt bei seiner synthetischen Herstellung oder der biotechnologischen Gewinnung teilweise in Form von sehr feinteiligen Pulvern, die teilweise aus langen gelben Nadeln bestehen, an. In dieser Form weist das Riboflavin sehr schlechte Handlings- und Fließeigenschaften auf.

Das feinteilige Pulver stäubt u.a. erheblich, weist eine sehr geringe Schüttdichte auf (meist unter 0,2 g/ml), lädt sich leicht elektrostatisch auf, zeigt ein schlechtes Fließverhalten und läßt sich daher nur mit großen Problemen weiterverarbeiten. Ein weiterer gravierender Nachteil des feinteiligen Pulvers ist es, daß es sich praktisch nicht zur Herstellung von Tabletten eignet, deren Riboflavinanteil 25 Gew.-% übersteigt (vgl. Bühler 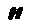 Vademecum for Vitamin Formulations", Wissenschaftliche Verlagsgesellschaft, Stuttgart, Seiten 98 bis 99).

Um diese Probleme zu lösen, wurden in der Vergangenheit Verfahren entwickelt, Riboflavin mit oder ohne Granulierhilfsmittel zu granulieren, um so ein Produkt zu erhalten, welches akzeptable Fließ- und Kompressionseigenschaften aufweist.

So beschreibt EP-A-0 219 276 Riboflavingranulate, die 90 bis 99 Gew.-% des Vitamins und zusätzlich ein Bindemittel enthalten.

EP-A-0 457 075 beschreibt ein Verfahren zur Herstellung von rieselfähigen, nichtstaubenden bindemittelfreien Riboflavinsprühgranulaten.

Obwohl sich diese Granulate verfahrenstechnisch gut für die Weiterverarbeitung eignen - sei es zur Direkttablettierung, zur Bereitung anderer, Riboflavin enthaltender Arzneimittelzubereitungen oder zur Herstellung von Vitamin B₂ enthaltenden Lebens- und Futtermitteln -, so ist häufig die Freisetzungsrate von Riboflavin, insbesondere in den mit diesen Granulaten hergestellten Tabletten, nicht immer zufriedenstellend.

Es war daher die Aufgabe der Erfindung ein Verfahren zu entwickeln, mit dessen Hilfe man auf technisch einfache Weise Riboflavingranulate herstellen kann, die sich einerseits gut für die Weiterverarbeitung, beispielsweise zur Direkttablettierung, eignen und die andererseits in den damit hergestellten Tabletten eine gute Freisetzungsrate an Riboflavin gewährleisten.

Diese Aufgabe wurde gelöst durch ein Verfahren zur Herstellung von Riboflavingranulaten oder Riboflavinmikrogranulaten mit einem Gehalt an Riboflavin von 90 bis 99,5 Gew.-% und mit einem Partikelgrößenbereich von 50 bis 450 µm, dadurch gekennzeichnet, daß man bei der Granulierung mindestens einen Hilfsstoff, ausgewählt aus der Gruppe, bestehend aus Alkali- und Erdalkalihalogeniden, Alkali- und Erdalkalicarbonaten, Alkali- und Erdalkalihydrogencarbonaten, Alkali- und Erdalkaliphosphaten, quervernetzter Cellulose und Cellulosederivate und quervernetztem Polyvinylpyrrolidon in Mengen von 0,5 bis 10 Gew.-%, alle Gew.-%-Angaben bezogen auf das Trockenprodukt, zusetzt.

Als Granulierverfahren im Rahmen der Erfindung eignen sich prinzipiell alle dem Fachmann bekannten Methoden zur Herstellung von Granulaten, wobei insbesondere die Feuchtgranulierung zu nennen ist. Dazu gehören u.a. die in EP-A-0 457 075, EP-A-0 497 177 und EP-A-0 219 276 genannten Granulierverfahren, basierend auf Sprühwirbelschichttrocknung und Sprübtrocknung.

Vorteilhaft gestaltet sich das erfindungsgemäße Verfahren, wenn man eine wäßrige oder Wasser enthaltende Suspension, bevorzugt eine rein wäßrige Suspension von Riboflavin zusammen mit mindestens einem Hilfsstoff, ausgewählt aus der Gruppe, bestehend aus Alkali- und Erdalkalihalogeniden, Alkali- und Erdalkalicarbonaten, Alkali- und Erdalkalihydrogencarbonaten, Alkali- und Erdalkaliphosphaten, quervernetzter Cellulose und Cellulosederivate und quervernetztem Polyvinylpyrrolidon einer Sprühtrocknung oder Sprühwirbelschichttrocknung, insbesondere einer agglomerierenden Sprühtrocknung, zuführt.

Unter einer Wasser enthaltenden Suspension von Riboflavin ist beispielsweise eine Suspension von Riboflavin in einem nicht zu hoch siedenden Lösungsmittel zu verstehen, wenn dieses Lösungsmittel zusätzlich Wasser enthält. Der Wassergehalt in der Suspension sollte dann mindestens 10 Gew.-% betragen. Als Lösungsmittel kommen insbesondere wassermischbare Lösungsmittel, wie beispielsweise C₁-C₄-Alkohole, in Betracht.

Bei den erfindungsgemäß verwendeten Hilfsstoffen handelt es sich um folgende Stoffklassen:
Alkali- und Erdalkalihalogenide wie z.B. NaCl, KCl, MgCl₂, CaCl₂, NaF, KF, NaJ, KJ;
Alkali- und Erdalkalicarbonate sowie Alkali- und Erdalkalihydrogencarbonate wie z.B. Na₂CO₃, K₂CO₃, CaCO₃, MgCO₃, NaHCO₃, KHCO₃;
Alkali- und Erdalkaliphosphate wie z.B. Na₃PO₄, Ca₃(PO₄)₂, CaHPO₄;
quervernetzte Cellulose und Cellulosederivate wie z.B. quervernetzte Natriumcarboxymethylcellulose (Fa. FMC Corp., USA);
quervernetztes Polyvinylpyrrolidon wie Z.B. Kollidon® CL, Kollidon® CL-M und Crospovidon® M (BASF Aktiengesellschaft).

Bevorzugte Hilfsstoffe für die erfindungsgemäß hergestellten Granulate sind Verbindungen, ausgewählt aus der Gruppe, bestehend aus Natriumchlorid, Kaliumchlorid, Natriumcarbonat, Kaliumcarbonat, Natriumhydrogencarbonat, quervernetzter Natriumcarboxymethylcellulose und quervernetztem Polyvinylpyrrolidon.

Zur Durchführung der Granulierung mittels Sprühtrocknung geht man im allgemeinen so vor, daß man eine wäßrige oder Wasser enthaltende Suspension einer Mischung aus Riboflavin und mindestens einem Hilfsstoff, ausgewählt aus der Gruppe, bestehend aus Alkali- und Erdalkalihalogeniden, Alkali- und Erdalkalicarbonaten, Alkali- und Erdalkalihydrogencarbonaten, Alkali- und Erdalkaliphosphaten, quervernetzter Cellulose und quervernetztem Polyvinylpyrrolidon nach Maßgabe der Trocknungsgeschwindigkeit in einem Sprühtrockner versprüht.

Dabei wird der Riboflavinsuspension zunächst mindestens einer der o.g. Hilfsstoffe - als Feststoff oder in Form einer wäßrigen Suspension - zugegeben und mit geeigneten Rühr- oder Dispergiermaschinen untergemischt.

Die Suspension mit einem Gehalt an Riboflavin von 5 bis 40 Gew.-%, bevorzugt 15 bis 30 Gew.-% und einem Gehalt an mindestens einem Hilfsstoff von 0,05 bis 4,5 Gew.-%, bevorzugt 0,15 bis 3,5 Gew.-% wird mit einer geeigneten Pumpe zum Zerstäuber des Sprühturms gefördert.

Die Zerstäubung im Sprühturm kann dabei sowohl mit Druckdüsen, Zweistoffdüsen oder Zentrifugalzerstäubern erfolgen. Bei viskosen Medien wird die Druckdüsenzerstäubung bevorzugt.

Das zum Trocknen der zerstäubten Riboflavintröpfchen in den Trockner eintretende Heizgas (Luft oder Inertgas) hat eine Eintrittstemperatur zwischen 120°C und 250°C, bevorzugt zwischen 150 und 200°C.

Produkt und Gas können im Trockner im Gleichstrom, Gegenstrom oder Mischstrom geführt werden, bevorzugt sind jedoch Gleichstromtürme.

Das getrocknete Granulat kann anschließend am Trocknerkonus ausgetragen oder mit dem Gasstrom mitgeführt und in einem Zyklon oder Filter abgeschieden werden.

Besonders bevorzugt arbeitet man mit einem Sprühtrockner mit integriertem Fließbett (Wirbelbett), wie er in Chem. Ing. Tech. 59 (1987) Nr. 2, Seite 112 bis 117 beschrieben wird. Dieses Verfahren wird auch agglomerierende Sprühtrocknung bezeichnet.

Bei dieser Trocknungsvariante können u.a. Produkte mit besseren Handlingseigenschaften hergestellt werden. Bei diesem Typ von Trocknern ist ein Wirbelbett an den Trockner angeflanscht und der Feinteil der Granulate wird in den Turm zurückgeführt, wo die Staubpartikel dort als Agglomerationskeime dienen. Diese Trockner werden häufig als FSD (Fluid Spray Dryer), SBD (Spray Bed Dryer) oder MSD (Multi Stage Dryer) bezeichnet.

Zur Durchführung der agglomerierenden Sprühtrocknung, die vorteilhafterweise kontinuierlich erfolgt, geht man im allgemeinen so vor, daß man
a) eine wäßrige Suspension einer Mischung aus 5 bis 40 Gew.-%, bevorzugt 15 bis 30 Gew.-%, Riboflavin und 0,05 bis 4,5 Gew.-%, bevorzugt 0,15 bis 3,5 Gew.-%, mindestens eines Hilfsstoffs, ausgewählt aus der Gruppe, bestehend aus Alkali- und Erdalkalihalogeniden, Alkali- und Erdalkalicarbonaten, Alkali- und Erdalkalihydrogencarbonaten, Alkali- und Erdalkaliphosphaten, quervernetzter Cellulose und Cellulosederivate und quervernetztem Polyvinylpyrrolidon nach Maßgabe der Trocknungsgeschwindigkeit kontinuierlich in den Trockner, versprüht,
b) das gebildete Trockenpulver als Granulat in einem, dem Trockner angeschlossenen und auf 20 bis 120°C, bevorzugt auf 40 bis 80°C, gehaltenen Wirbelbett auffängt,
c) die Granulatpartikel mit einem Gehalt an Riboflavin von 90 bis 99,5 Gew.-% und einem Gehalt an mindestens einem der unter a) genannten Hilfsstoffe von 0,5 bis 10 Gew.-%, jeweils bezogen auf das Trockenprodukt, nach geeigneter Verweilzeit aus dem Wirbelbett kontinuierlich abzieht,
d) die abgetrennten Granulatpartikel gegebenenfalls durch eine geeignete Vorrichtung in Partikelfraktionen auftrennt und
e) die feinerteiligen Granulatpartikel und/oder die durch Vermahlen von größeren Granulatpartikeln erhaltenen feinerteiligen Partikel in den Sprühtrockner als Agglomerationskeime zurückführt.

Ferner können die von der Abluft mitgeführten und im Zyklon oder Staubfilter abgeschiedenen feinteiligen Granulatpartikel in den Sprühtrockner als Agglomerationskeime zurückgeführt werden.

Die Herstellung von Riboflavingranulaten mittels Sprühwirbelschichttrocknung kann sowohl diskontinuierlich als auch kontinuierlich durchgeführt werden. Bevorzugt arbeitet man, analog der agglomerierenden Sprühtrocknung, kontinuierlich.

Zur Durchführung der kontinuierlichen Sprühwirbelschichttrocknung geht man im allgemeinen so vor, daß man
a) Riboflavin in Form eines Riboflavintrockenpulvers oder eines Sprüh- oder Mikrogranulates alleine oder zusammen mit einem Hilfsstoff, ausgewählt aus der Gruppe, bestehend aus Alkali- und Erdalkalihalogeniden, Alkali- und Erdalkalicarbonaten, Alkali- und Erdalkalihydrogencarbonaten, Alkali- und Erdalkaliphosphaten, quervernetzter Cellulose und Cellulosederivate und quervernetztem Polyvinylpyrrolidon in einem auf 20 bis 120°C bevorzugt 40 bis 80°C, gehaltenen Wirbelbett zum Anfahren des Prozesses vorlegt,
b) hierzu die wäßrige Suspension einer Mischung aus 5 bis 40 Gew.-%, bevorzugt 15 bis 30 Gew.-%, Riboflavin und 0,05 bis 4,5 Gew.-%, bevorzugt 0,15 bis 3,5 Gew.-%, mindestens eines Hilfsstoffs, ausgewählt aus der Gruppe, bestehend aus Alkali- und Erdalkalihalogeniden, Alkali- und Erdalkalicarbonaten, Alkali- und Erdalkalihydrogencarbonaten, Alkali- und Erdalkaliphosphaten, quervernetzter Cellulose und quervernetztem Polyvinylpyrrolidon nach Maßgabe der Trocknungsgeschwindigkeit kontinuierlich in die Wirbeischicht einsprüht,
c) die Granulatpartikel mit einem Gehalt an Riboflavin von 90 bis 99,5 Gew.-% und einem Gehalt an mindestens einem der unter a) genannten Hilfsstoffe von 0,5 bis 10 Gew.-%, jeweils bezogen auf das Trockenprodukt, nach geeigneter Verweilzeit aus dem Wirbelbett kontinuierlich abzieht,
d) die abgetrennten Granulatpartikel gegebenenfalls durch eine geeignete Vorrichtung in Partikelfraktionen auftrennt und
e) die feinerteiligen Granulatpartikel und/oder die durch Vermahlen von größeren Granulatpartikeln erhaltenen feinerteiligen Partikel in den Granulierungsprozeß zurückführt.

Das zur Bildung des Wirbelbettes verwendete Wirbelgas hat im allgemeinen eine Eintrittstemperatur von 60 bis 250°C, vorzugsweise 140 bis 185°C, und eine Austrittstemperatur von 40 bis 140°C, vorzugsweise 60 bis 85°C, wodurch sich Temperaturen von etwa 20 bis 120°C, vorzugsweise 40 bis 80°C, insbesondere 60 bis 80°C, im Wirbelbett ergeben.

Im Gleichgewichtszustand des Prozesses ist der Mengenstrom an Wertprodukt, der aus dem Prozeß ausgeschleust wird, so groß wie der, den Sprühdüsen zugeführte Riboflavin/Hilfsmittel-Mengenstrom, auf Basis Trockensubstanz.

Bei der Durchführung der erfindungsgemäßen Granulierverfahren ergeben sich im allgemeinen - je nach Korngrößensteuerung - folgende Partikelgrößenfraktionen:
1. etwa 5 bis 95 %, bevorzugt 30 bis 70 %, im Partikelgrößenbereich bis 100 µm,
2. etwa 25 bis 85 %, bevorzugt 30 bis 70 %. im Partikelgrößenbereich von 100 bis 300 µm.
3. etwa 1 bis 30 % im Partikelgrößenbereich > 300 µm,
   wobei die Summe der drei Partikelgrößenfraktionen 100 % ergibt.

Die Eigenschaften des Endproduktes werden nicht nur durch den Trocknungsprozeß sondern auch durch die Form und Größe der Riboflavinkristalle beeinflußt.

Vorteilhaft für den Agglomerationsprozeß ist eine mittlere Kristallgröße zwischen 0,1 und 10 µm, bevorzugt 0,3 und 5 µm, besonders bevorzugt zwischen 0,5 und 3 µm. Die entsprechende Kristallgröße kann durch Mahlung, bevorzugt Naßmahlung der Riboflavinsuspension, beispielsweise mittels Rührwerkskegelmühlen, erreicht werden. Die Suspension wird dabei in Kreis- oder Passagenfahrweise durch die Mühle gepumpt. Die rotierenden Rührwerkzeuge in der Mühle erzeugen dabei die Scherbeanspruchung zwischen Mahlgut und den Mahlkörpern. Zur Vermeidung von Abrieb der Mahlkörper ist es sinnvoll, besonders harte Mahlkörper einzusetzen (z.B. yttriumstabilisierte Keramikmahlkörper).

Gegenstand der Erfindung sind auch Riboflavingranulate oder Riboflavinmikrogranulate, enthaltend 90 bis 99,5 Gew.-%, bevorzugt 93 bis 99 Gew.-%, Riboflavin und 0,5 bis 10 Gew.-%, bevorzugt 1 bis 7 Gew.-% ,mindestens eines Hilfsstoffs, ausgewählt aus der Gruppe, bestehend aus Alkali- und Erdalkalihalogeniden, Alkali- und Erdalkalicarbonaten, Alkali- und Erdalkalihydrogencarbonaten, Alkali- und Erdalkaliphosphaten, quervernetzter Cellulose und Cellulosederivate und quervernetztem Polyvinylpyrrolidon, wobei sich die Gew.-% Angaben jeweils auf das Trockenprodukt beziehen.

Die bevorzugten Riboflavingranulate oder Riboflavinmikrogranulate enthalten mindestens einen Hilfsstoffs, ausgewählt aus der Gruppe, bestehend aus Natriumchlorid, Kaliumchlorid, Natriumcarbonat, Kaliumcarbonat, Natriumhydrogencarbonat, quervernetzter Natriuncarboxymethylcellulose und quervernetztem Polyvinylpyrrolidon und zeigen einen Partikelgrößenbereich von 50 bis 450 µm, bevorzugt 100 bis 300 µm aus feinteiligem Riboflavin.

Die erfindungsgemäßen Riboflavingranulate oder Riboflavinmikrogranulate eignen sich in vorteilhafter Weise zur Herstellung von festen pharmazeutischen Darreichungsformen, bevorzugt von Tabletten, insbesondere solcher, die mittels Direkttablettierverfahren hergestellt werden.

Gegenstand der Erfindung sind somit auch feste Darreichungsformen, die die o.g. erfindungsgemäßen Riboflavingranulate enthalten. Darunter versteht man u.a. Tabletten, Mikrotabletten, Dragees, Pastillen, Kapseln oder Pellets, bevorzugt Tabletten oder Mikrotabletten, besonders bevorzugt direkttablettiertes Riboflavin.

Der Gehalt an Riboflavin in den Tabletten liegt im Bereich von 1 bis 100 mg, bevorzugt 5 bis 75 mg, besonders bevorzugt im Bereich von 5 bis 50 mg.

Bei der Herstellung der festen Darreichungsformen können selbstverständlich auch weitere Hilfsstoffe zugesetzt werden.

Dies können u.a. sein:
Füllstoffe und Bindemittel wie z.B. Lactose, Calciumphosphate, Cellulose und Cellulosederivate, Stärke und Stärkederivate, partiell verseiftes Polyvinylacetat, Zuckeralkohole, Zucker, Fette, Wachse;
Sprengmittel wie z.B. Kollidon® CL (Fa. BASF), Na-Carboxymethylstärke, Na-Carboxymethylcellulose;
Gleit- und Schmiermittel wie z.B. Mg-stearat, Ca-behenat, Stearinsäure, PEG;
Fließregulierungsmittel wie z.B. hochdisperses Siliciumdioxid;
Filmbildner wie z.B. Polyacrylate und Polymethacrylate (Eudragittypen), Copolymere auf Basis von Acrylatderivaten, Hydroxypropylmethylcellulose, Hydroxypropylcellulose, Celluloseacetat, Celluloseacetatphthalat und andere magensaftresistente Überzugsmaterialien;
Feuchthaltemittel wie z.B. Glycerin, Propylenglykol, Sorbitol, Mannitol, Polyethylenglykole sowie
   Weichmacher, Farbstoffe, Tenside, Salze, Dispergierhilfsmittel.

Die unter Verwendung der erfindungsgemäßen Riboflavin-haltigen Granulate hergestellten festen pharmazeutischen Darreichungsformen zeichnen sich u.a. durch eine hohe in-vitro Freisetzungsrate an Riboflavin aus. So liegt die Freisetzungsrate, gemessen nach dem USP XXII Paddle-Modell, bei mindestens 75 Gew.-% Riboflavin, bevorzugt bei größer 80 Gew.-%, besonders bevorzugt bei 83 bis 95 Gew.-%, Riboflavin nach 60 min.

In den nachfolgenden Beispielen wird die Herstellung und Verwendung der erfindungsgemäßen Riboflavingranulate näher erläutert.

### Herstellung der Riboflavin-haltigen Granulate

### Beispiel 1

Eine 20 gew.-%ige, wäßrige Riboflavinsuspension wurde mittels einer Kugelmühle auf eine mittlere Partikelgröße von 1 µm gemahlen, mit 5 Gew.-% NaHCO₃ (bezogen auf den Gesamtfeststoffgehalt) versetzt und homogenisiert. Anschließend wurde die Suspension in einem FSD-Trockner (Niro, Typ 12,5) mit 130 bar versprüht. Die Eintrittstemperatur des Trocknungsgases in den Sprühturm betrug 190°C, die Turmaustrittstemperatur 60°C. Die zulufttemperatur für das interne Fließbett betrug ebenfalls 60°C. Die Abscheidung des Feingutes erfolgte in einem 40-m²-Schlauchfilter, wobei das Feingut in den Sprühturm zurückgeführt wurde. Das so hergestellte Mikrogranulat hatte einen Wassergehalt von weniger als 1,5 Gew.-% und eine mittlere Teilchengröße von ca. 120 µm.

### Beispiele 2 bis 4

Analog Beispiel 1 wurden Riboflavingranulate hergestellt, die neben 95 Gew.-% Riboflavin 5 Gew.-% der folgenden Hilfsstoffe enthielten (die Gew.-% Angaben jeweils bezogen auf auf das Trockenprodukt):

### Beispiel 2:

5 Gew.-% NaCl,
   mittlere Teilchengröße: 110 µm;

### Beispiel 3:

5 Gew.-% Kollidon® CL-M (Fa. BASF Aktiengesellschaft)
   mittlere Teilchengröße: 120 µm;

### Beispiel 4:

5 Gew.-% AcDiSol® (Fa. FMC Corp.)
   mittlere Teilchengröße: 115 µm.

### Beispiel 5

Eine 17 gew. -%ige, wäßrige Riboflavinsuspension wurde mittels einer Kugelmühle auf eine mittlere Partikelgröße von 2 µm gemahlen, mit 5 Gew.-% quervernetzter Na-Carboxymethylcellulose (bezogen auf den Gesamtfestoffgelt) versetzt und homogenisiert. Die Suspension wurde anschließend in einem Laborsprühtrockner (Fa. Niro, Typ Minor) mit einer Zweistoffdüse bei 4 bar zerstäubt. Die Turmeintrittstemperatur betrug 190°C, die Turmaustrittstemperatur 62°C. Das Riboflavinpulver wurde in einem Zyklon von der Trocknungsluft abgeschieden. Das so hergestellte Pulver hatte einen Wassergehalt von 0,5 Gew.-% und eine mittlere Teilchengröße von 70 µm.

### Herstellung Riboflavin-haltiger Tabletten

### Beispiele 6 bis 9

266,7 g Lactose (Tablettose®, Fa. Meggle), 134,3 g mikrokristalline Cellulose (Avicel® PH 102, Fa. FMC), 10 g hochdisperses SiO₂ (Aerosil®, Fa. Degussa) sowie 2,5 g quervernetzte Na-Carboxymethylcellulose (AcDiSol®, Fa. FMC Corp.) wurden 10 Minuten homogenisiert und durch ein Sieb von 0,8 mm Maschenweite gesiebt. Anschließend wurden 3,3 g Magnesiumstearat und 83,3 g Riboflavingranulat, hergestellt nach einem der Beispiele 1 bis 4, zugegeben und die Mischung erneut homogenisiert. Die fertige Tablettenmischung wurde auf einer Rundläuf er Tablettenpresse Korsch PH 106 bei einer Tablettengeschwindigkeit von 20 U/min und einer Preßkraft von 10 kN zu 8 mm Tabletten verpreßt.

### Zusammensetzung der Tablette:

50 mg Vitamin B₂ mit Hilfsstoff, gemäß Tabelle 1
160 mg Tablettose®
80,5 mg Avicel® PH 102
1,5 mg AcDiSol®
6,0 mg Aerosil®
2,0 mg Mg-Stearat

### Beispiel 10

266,7 g Tablettose®, 134,3 g Avicel® PH 102, 10 g Aerosil® sowie 2,5 g AcDiSol® wurden 10 Minuten homogenisiert und durch ein Sieb von 0,8 mm Maschenweite gesiebt. Anschließend wurden 3,3 g Magnesiumstearat und 83,3 g Riboflavingranulat, hergestellt nach Beispiel 5, zugegeben und die Mischung erneut homogenisiert. Die fertige Tablettenmischung wurde auf einer Rundläuf er Tablettenpresse Korsch PH 106 bei einer Tablettengeschwindigkeit von 20 U/min und einer Preßkraft von 10 kN zu 8 mm Tabletten verpreßt.

### Zusammensetzung der Tablette:

50 mg Vitamin B₂ mit Hilfsstoff, gemäß Tabelle 1
160 mg Tablettose®
80,5 mg Avicel® PH 102
1,5 mg AcDiSol®
6,0 mg Aerosil®
2,0 mg Mg-Stearat

### Beispiel 11 (Vergleichsbeispiel)

266,7 g Tablettose® (Fa. Meggle), 134,3 g Avicel® PH 102 (Fa. FMC) sowie 10 g Aerosil® (Fa. Degussa) sowie 2,5 g quervernetzter Cellulose (AcDiSol®, Fa. FSM) wurden 10 Minuten homogenisiert und durch ein Sieb von 0,8 mm Maschenweite gesiebt. Anschließend wurden 3,3 g Magnesiumstearat und 83,3 g Riboflavingranulat (hergestellt gemäß Beispiel 1, jedoch ohne Zusatz eines Hilfsstoffs) zugegeben und die Mischung erneut homogenisiert. Die fertige Tablettenmischung wurde auf einer Rundläuf er Tablettenpresse Korsch PH 106 bei einer Tablettengeschwindigkeit von 20 U/min und einer Preßkraft von 10 kN zu 8 mm Tabletten verpreßt.

### Zusammensetzung der Tablette:

50 mg Vitamin B₂ ohne Hilfsstoff
160 mg Tablettose®
80,5 mg Avicel® PH 102
1,5 mg AcDiSol®
6,0 mg Aerosil®
2,0 mg Mg-Stearat

Die Freisetzungsrate von Riboflavin aus den nach den Beispielen 6 bis 11 hergestellten Tabletten wurde nach dem Dissolution Test, gemäß USP XXII in 900 ml 0,1 N HCl bei 75 U/min und 37°C und UV-Detektion bei 267 nm ermittelt.

Zur besseren Differenzierung der Meßergebnisse gibt der in Tabelle 1 angegebene Freisetzungswert die Freisetzung von Riboflavin nach bereits 30 min wieder.

**Tabelle 1**

| Tablette gemäß Beispiel | Hilfsstoff | Freisetzungsrate (nach 30 min) |
|---|---|---|
| | | |
| 6 | NaHCO₃ | 86 % |
| 7 | NaCl | 83 % |
| 8 | Kollidon® CL | 88 % |
| 9 | AcDiSol® | 86 % |
| 10 | AcDiSol® | 88 % |
| | | |
| 11 (Vergleich) | - | 75 % |
| | | |

## Patentansprüche

1. Verfahren zur Herstellung von Riboflavingranulaten oder Riboflavinmikrogranulaten mit einem Gehalt an Riboflavin von 90 bis 99,5 Gew.-% und mit einem Partikelgrößenbereich von 50 bis 450 µm, dadurch gekennzeichnet, daß man bei der Granulierung mindestens einen Hilfsstoff, ausgewählt aus der Gruppe, bestehend aus Alkali- und Erdalkalihalogeniden, Alkali- und Erdalkalicarbonaten, Alkali- und Erdalkalihydrogencarbonaten, Alkali- und Erdalkaliphosphaten, quervernetzter Cellulose und Cellulosederivate und quervernetztem Polyvinylpyrrolidon in Mengen von 0,5 bis 10 Gew.-%, alle Gew.-%-Angaben jeweils bezogen auf das Trockenprodukt, zusetzt.

2. Verfahren nach Anspruch 1 unter Verwendung von 0,5 bis 10 Gew.-% mindestens eines Hilfsstoffs, ausgewählt aus der Gruppe, bestehend aus Natriumchlorid, Kaliumchlorid, Natriumcarbonat, Kaliumcarbonat, Natriumhydrogencarbonat, quervernetzter Natriumcarboxymethylcellulose und quervernetztem polyvinylpyrrolidon.

3. Verfahren nach einem der Ansprüche 1 oder 2, dadurch gekennzeichnet, daß man eine wäßrige Suspension, enthaltend 5 bis 40 Gew.-% Riboflavin und 0,05 bis 4,5 Gew.-% mindestens eines Hilfsstoffs, definiert gemäß einem der Ansprüche 1 oder 2, einer Sprühtrocknung oder Sprühwirbelschichttrocknung zuführt.

4. Verfahren nach Anspruch 3, dadurch gekennzeichnet, daß es sich bei der Sprühtrocknung oder Sprühwirbelschichttrocknung um ein kontinuierliches Verfahren handelt.

5. Verfahren nach Anspruch 4, dadurch gekennzeichnet, daß es sich bei der Sprühtrocknung um eine kontinuierliche agglomerierende Sprühtrocknung handelt, bei deren Durchführung man
a) eine wäßrige Suspension einer Mischung aus Riboflavin und mindestens einem Hilfsstoff, ausgewählt aus der Gruppe, bestehend aus Alkali- und Erdalkalihalogeniden, Alkali- und Erdalkalicarbonaten, Alkali- und Erdalkalihydrogencarbonaten, Alkali- und Erdalkaliphosphaten, quervernetzter Cellulose und Cellulosederivate und quervernetztem Polyvinylpyrrolidon nach Maßgabe der Trocknungsgeschwindigkeit kontinuierlich in den Trockner versprüht,
b) das gebildete Trockenpulver als Granulat in einem, dem Trockner angeschlossenen und auf 20 bis 120°C gehaltenen Wirbelbett auffängt,
c) die Granulatpartikel mit einem Gehalt an Riboflavin von 90 bis 99,5 Gew.-% und einem Gehalt an mindestens einem der unter a) genannten Hilfsstoffe von 0,5 bis 10 Gew.-%, jeweils bezogen auf das Trockenprodukt, nach geeigneter Verweilzeit kontinuierlich aus dem Wirbelbett abzieht,
d) die abgetrennten Granulatpartikel gegebenenfalls durch eine geeignete Vorrichtung in Partikelfraktionen auftrennt und
e) die feinerteiligen Granulatpartikel und/oder die durch Vermahlen von größeren Granulatpartikeln erhaltenen feinerteiligen Partikel in den Sprühtrockner als Agglomerationskeime zurückführt.

6. Verfahren nach Anspruch 4, dadurch gekennzeichnet, daß man zur Durchführung der kontinuierlichen Sprühwirbelschichttrocknung
a) Riboflavin in Form eines Riboflavintrockenpulvers oder eines Sprüh- oder Mikrogranulates alleine oder zusammen mit einem Hilfsstoff, ausgewählt aus der Gruppe, bestehend aus Alkali- und Erdalkalihalogeniden, Alkali- und Erdalkalicarbonaten, Alkali- und Erdalkalihydrogencarbonaten, Alkali- und Erdalkaliphosphaten, quervernetzter Cellulose und Cellulosederivate und quervernetztem Polyvinylpyrrolidon in einem auf 20 bis 120°C gehaltenen Wirbelbett zum Anfahren des Prozesses vorlegt,
b) hierzu die wäßrige Suspension einer Mischung aus Riboflavin und mindestens einem Hilfsstoff, ausgewählt aus der Gruppe, bestehend aus Alkali- und Erdalkalihalogeniden, Alkali- und Erdalkalicarbonaten, Alkali- und Erdalkalihydrogencarbonaten, Alkali- und Erdalkaliphosphaten, quervernetzter Cellulose und Cellulosederivate und quervernetztem Polyvinylpyrrolidon nach Maßgabe der Trocknungsgeschwindigkeit kontinuierlich in die Wirbelschicht einsprüht,
c) die Granulatpartikel mit einem Gehalt an Riboflavin von 90 bis 99,5 Gew.-% und einem Gehalt an mindestens einem der unter a) genannten Hilfsstoffe von 0,5 bis 10 Gew.-%, jeweils bezogen auf das Trockenprodukt, nach geeigneter Verweilzeit aus dem Wirbelbett kontinuierlich abzieht,
d) die abgetrennten Granulatpartikel gegebenenfalls durch eine geeignete Vorrichtung in Partikelfraktionen auftrennt und
e) die feinerteiligen Granulatpartikel und/oder die durch Vermahlen von größeren Granulatpartikeln erhaltenen feinerteiligen Partikel in den Granulierungsprozeß zurücktührt.

7. Verfahren nach einem der Ansprüche 1 bis 6, dadurch gekennzeichnet, daß man die Riboflavinpartikel vor der Granulierung bis zu einer Partikelgröße von 0,1 bis 10 µm vermahlt.

8. Riboflavingranulate oder Riboflavinmikrogranulate, enthaltend 90 bis 99,5 Gew.-% Riboflavin und 0,5 bis 10 Gew.-% mindestens eines Hilfsstoffs, ausgewählt aus der Gruppe, bestehend aus Alkali- und Erdalkalihalogeniden, Alkali- und Erdalkalicarbonaten, Alkali- und Erdalkalihydrogencarbonaten, Alkali- und Erdalkaliphosphaten, quervernetzter Cellulose und Cellulosederivate und quervernetztem Polyvinylpyrrolidon, wobei sich die Gew.-% Angaben jeweils auf das Trockenprodukt beziehen.

9. Riboflavingranulate oder Riboflavinmikrogranulate nach Anspruch 8, enthaltend mindestens einen Hilfsstoffs, ausgewählt aus der Gruppe, bestehend aus Natriumchlorid, Kaliumchlorid, Natriumcarbonat, Kaliumcarbonat, Natriunhydrogencarbonat, quervernetzter Natriumcarboxymethylcellulose und quervernetztem Polyvinylpyrrolidon.

10. Riboflavingranulate oder Riboflavinmikrogranulate nach einem der Ansprüche 8 oder 9 mit einem Partikelgrößenbereich von 50 bis 450 µm aus feinteiligem Riboflavin.

11. Riboflavingranulate oder Riboflavinmikrogranulate nach einem der Ansprüche 8 bis 10, hergestellt nach einem Verfahren, definiert gemäß einem der Ansprüche 1 bis 7.

12. Verwendung von Riboflavingranulaten oder Riboflavinmikrogranulaten, definiert gemäß Anspruch 8, zur Herstellung von festen pharmazeutischen Darreichungsformen.

13. Verwendung von Riboflavingranulaten oder Riboflavinmikrogranulaten nach Anspruch 12 zur Herstellung von Tabletten.

14. Tabletten, enthaltend Riboflavingranulate oder Riboflavinmikrogranulate, definiert gemäß einem der Ansprüche 8 bis 11.

15. Tabletten nach Anspruch 14 mit einem Riboflavingehalt von 1 bis 100 mg.

16. Tabletten nach einem der Ansprüche 14 oder 15 mit einer in-vitro-Freisetzungsrate nach USP XXII von mindestens 75 Gew.-% Riboflavin nach 60 min.
